# EUROPEAN PATENT SPECIFICATION

(11) **EP 0 991 390 B1**
(45) Date of publication and mention of the grant of the patent: **16.01.2002**
(21) Application number: 98931655.9
(22) Date of filing: 26.06.1998
(51) Int. Cl.: A61F 13/15, A61F 5/451

(54) **FAECAL COLLECTOR**
FÄKALIENAUFNAHMEBEHÄLTER
COLLECTEUR DE MATIERES FECALES

(30) Priority: 28.06.1997 EP 97110602; 28.06.1997 EP 97110603; 28.06.1997 EP 97110604
(43) Date of publication of application: 12.04.2000
(73) Proprietor: THE PROCTER & GAMBLE COMPANY, Cincinnati, Ohio 45202 (US)
(72) Inventor: PALUMBO, Gianfranco, D-61348 Bad Homburg (DE); D'ACCHIOLI, Vincenzo, D-65779 Kelkheim am Taunus (DE)
(74) Representative: Kohol, Sonia
(86) International application number: US9813359
(87) International publication number: WO9900086

(56) References cited:
- EP-A- 0 753 290
- GB-A- 1 078 588
- GB-A- 1 092 274
- GB-A- 2 116 849
- US-A- 3 577 989
- PATENT ABSTRACTS OF JAPAN vol. 096, no. 009, 30 September 1996 & JP 08 117261 A (BEPPU YOSHIO), 14 May 1996

## Description

### Field of the Invention

The present invention relates to a faecal management device having a softer tactile feel for increased wearer comfort and satisfaction.

### Background of the Invention

Faecal management devices are known articles of manufacture that are designed to be worn principally by incontinence sufferers and infants. Such faecal management devices are attached to the anal region of the wearer and are intended to entrap and immediately contain faecal material and other bodily discharges. As a consequence, these devices are functionally effective in eliminating the problem of smearing on the skin of the wearer; in lessening epidermal irritation; in preventing contamination of articles such as clothing and bedding; and even in preventing the soiling of the carers themselves. Nevertheless, a problem often encountered during the use of such faecal management devices is that the constituent material of the outer surface of the devices tends to cause discomfort and in some cases, extreme discomfort to, for example, the bedridden wearer or to the infant.

Typically, the faecal management devices are made from a plastic material. For instance, US 3,577,989 details a disposable elimination-trapping bag for incontinence sufferers including a sack having an open-top portion, and a flange secured to the sack around the open-top portion. The elimination trapping bag is made from a light thin plastic. US 4,784,656 describes a receptacle for collecting faecal matter from incontinence sufferers. The faecal collector comprises a gasket, conduit means and a receptacle. The receptacle and conduit means are each formed from two sheets of odour barrier thermoplastic film that are heat sealed along their side edges, respectively. In a further embodiment, the receptacle and conduit means may also be made of any suitable fluid impervious, odour-barrier film or plastic. GB 2 152 387 describes a faecal collector for incontinence sufferers comprising a collection bag and a ring according to the preamble of claim 1. The collection bag is preferably formed from a single sheet of odour-barrier thermoplastic film folded along a vertical midline to provide a pair of continuous panels. In another embodiment, the collection bag may be formed of any suitable thermoplastic film or film laminate. EP 0 245 064 describes a faecal incontinence bag having flexible front and rear walls secured together around their periphery. The front wall has a hole for entry of the matter discharged by the wearer. Normally, the front and rear walls are made of synthetic plastic material such as PVC or multilayer films with high odour barrier properties. The surface of the front wall for contact with the wearer may be provided with a so-called "comfort layer", i.e., a layer of perforated or porous plastic material to prevent the bag from sticking to the wearer.

Plastic material, while functionally acceptable, is endowed with certain characteristics that are unsatisfactory and disagreeable to the wearer. The feel or texture of plastic material is particularly disturbing from the point of view of skin healthiness. Typically, the skin of incontinence sufferers and infants is especially sensitive and needs to be treated gently and with care. It has been recognised that the rubbing of plastic material from a faecal management device against the body of a wearer during wear can lead to occlusion, create hot clammy conditions and stickiness, reddening, skin rashes and perhaps, even lead to a more severe skin irritation and ultimately to the disregarding of the device. Therefore, a real consumer need can be identified for a faecal management device with superior cushioning qualities and a softer tactile feel.

The present invention addresses this need by providing the outer surface of the faecal management device with a hydrophobic fibrous layer, which is preferably a nonwoven layer. It has been found that the presence of this fibrous hydrophobic layer is uniquely advantageous and greatly enhances the softness of the faecal management device without adversely affecting its functionality. Furthermore, the faecal management device is aesthetically more pleasing, produces less crinkle during use and results in a high level of wearer and carer satisfaction in relation to skin healthiness.

In another aspect of the present invention, the faecal management device with its hydrophobic fibrous outer surface outer layer can be advantageously used with a disposable diaper. In particular the presence of the hydrophobic fibrous outer ensures that any fluid which may contact the outer surface is repelled and is hence absorbed by the diaper and is not absorbed by the outer layer itself. The prior art is actually silent on such a combination. JP 08-117,261 only teaches a diaper having a bag-like structure made of waterproof material at least at the anus facing portion, in which the bag-like structure has a slit or a hole having an adhesive coated on the periphery at the hip-contacting surface. The document, however, does not disclose two separate entities that work synergistically to isolate the skin of the wearer from the absorbent material of the diaper. In this manner, the constituent material of the outer surface of the faecal management device can be capitalised upon to reduce not only the problem of occlusion and epidermal irritations, but also contribute greatly to improved skin healthiness and lead to very satisfied wearers.

### Summary of the Invention

A faecal management device (10) constructed in accordance with the present invention comprises a bag (11) having an aperture (21) and an anatomically shaped flange (12) surrounding the aperture for adhesive attachment to the perianal area of the wearer. The anatomically-shaped flange is attached to the bag. The bag (11) comprises a wall material that has an outer surface (30) and an inner surface (15). The outer surface (30) is provided with a hydrophobic fibrous layer, wherein said fibres have a wetting time of a least 1 hour. Preferably said layer is a nonwoven layer. The inner surface preferably comprises a plastic film layer. The plastic film is preferably permeable to air and to vapour.

In a preferred embodiment of the present invention, the hydrophobic fibrous layer (30), which is preferably a nonwoven layer and the plastic film layer (15) are in the form of a laminate.

In another aspect of the present invention, the present faecal management device is used in combination with a disposable diaper.

### Brief description of the drawings

It is believed that the invention will be better understood from the foregoing description in conjunction with the accompanying drawings in which:
Figure 1 is a perspective view of a faecal management device according to the present invention;

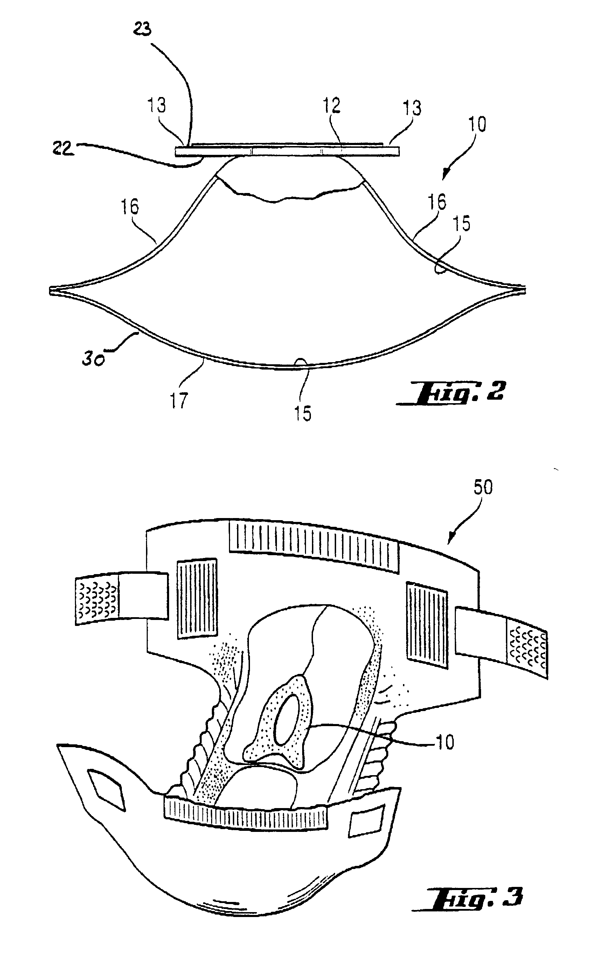

Figure 2 is a cross sectional view of the faecal management device as displayed in Figure 1;
Figure 3 shows a perspective view of the faecal management device in conjunction with a disposable diaper; and
Figure 4 is a partially cut-away perspective view of a disposable diaper embodying the present invention.

### Detailed description of the Invention

Typically faecal management devices (10) comprise a bag (11) having an aperture (21) and a flange (12) surrounding the aperture (21) for preferably adhesive attachment to the perianal area of a wearer. Any faecal management device (10) known in the art can be provided according to the present invention.

The bag (11) as used herein is a flexible receptacle for the containment of excreted faecal matter. The bag (11) can be provided in any shape or size depending on the intended use thereof, i.e. whether the device is intended for bedridden patients or active patients suffering from incontinence or requiring an artificial bowel or for infants. For example elongated bags which are principally tubular or rectangular are typically utilised by bedridden patients and elderly incontinence sufferers. For more active wearers whether infants or adults, the faecal management device should preferably be anatomically shaped such that the device follows the contours of the body and can be worn inconspicuously by the wearer under normal garments.

Particularly, preferred shapes are flat circular type bags, cone shaped bags, truncated cone shaped bags and pyramidal or truncated pyramidal shaped bags. In a most preferred embodiment of the present invention, the bag (11) has a substantially truncated cone shape. Typically the bags will have a wearer facing portion (16) and a garment facing portion (17). The wearer facing portion (16) of the faecal management device (10) is disposed adjacent the buttocks of the wearer. As such, the wearer facing portion (16) amply covers the buttocks of the wearer and does not hang between the thighs of the wearer.

In addition, the bag (11) is preferably shaped to allow at least partial insertion and retention of the bag in-between the buttocks of the wearer and thereby ensure good contact between the flange and the skin of the wearer. For example the bag (11) may be provided with a neck portion or conduit.

The bag (11) is preferably designed to provide sufficient volume for faecal material under a variety of wearing conditions, also when worn by a freely moving, i.e. not bedridden wearer. Sitting on the bag, for example, will result in a largely reduced volume in some areas of the bag. Thus, the bag is preferably shaped to provide sufficient volume in areas which are not subjected to much pressure in wearing conditions such as sitting.

The bag (11) is designed to safely contain any entrapped material, typically it will be liquid impermeable, yet it may be breathable. The bag (11) is designed of sufficient strength to withstand rupture in use, also when pressure on the bag (11) is exerted in typical wearing conditions, such as sitting.

According to the present invention, depending on the shape of the bag (11) required, the bag (11) may be provided from a unitary piece of material or from a number of separate pieces of material, which may be identical or different and which are sealed at their respective peripheries.

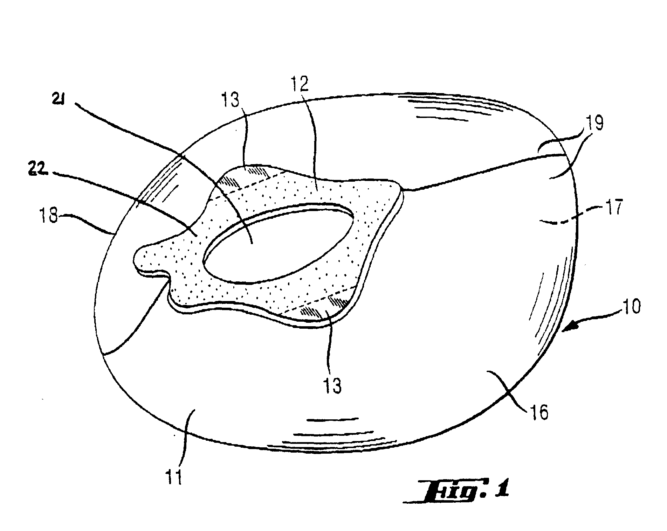

In one preferred embodiment the bags herein have a wearer facing portion (16) and a garment facing portion (17) which comprise separate pieces of material. The wearer facing portion (16) and the garment facing portion (17) are sealed at the periphery of the bag (11), thus creating a bag peripheral rim (18). As is visible from Figure 1, the wearer facing portion (16) of the bag (11) may comprise two further sections (19), which are secured to each other by means known to the man skilled in the art, such as adhesive, thermobonding or pressure bonding in order to provide the desired bag configuration. Said rim (18) may also be inside the bag (11), thus being coextensive with the inner surface (15) of the bag (11) rather than with the outer surface (30) of the bag (11). Preferably the bag is asymmetrical to the transverse axis, so that the distance measured in the longitudinal direction from the centre of the aperture (21) to the front end of the bag (11) is shorter than the (12) distance measured to the rear end of the bag (11).

According to the present invention the bag (11) can comprise one or multiple layers, preferably two or three layers. The layer on the inside of the bag, which will typically at least partially come in contact with faecal material is called the inner layer (15). The outermost layer of the bag, which will typically at least partially come in contact with the skin to the wearer and the garments of the wearer, is called the outer layer (30).

According to the present invention an essential feature of the bag material is that the outer layer (30) of the bag is a fibrous layer, wherein the fibres are hydrophobic such that said fibres have a wetting time of at least 1 hour, preferably at least 6 hours and most preferably at least 24 hours. It has been surprisingly found that the provision of such a hydrophobic fibrous outer layer provides the bag with particularly pleasing sensation to the skin and in particular when utilised in conjunction with a diaper ensures that fluid is not absorbed by the outer layer of the wall material of the faecal management device but is absorbed within the core of the diaper.

The hydrophobicitiy of the fibrous outer layer is determined according to the liquid absorbency time test as per the ASTM -D 1117-80 method.

According to the present invention any fibre which meets the hydrophobicity requirements can be usefully employed as the outer layer. Suitable fibres include fibres which meet the hydrophobicity requirements per se such as polyester fibres, polypropylene fibres, polyethylene fibres, polyamide fibres and mixtures thereof. Alternatively fibres which have been treated by the utilisation of a particular finishing treatment for example an oil, in order to provide them with the required hydrophobicity requirements may also be effectively utilised herein. Moreover mixtures of hydrophobic fibres or treated hydrophobic fibres may also be utilised. The fibrous outer layer may be provided as a woven or a nonwoven layer, preferably a nonwoven.

The additional layers of the bag material may be provided from any material, preferably so that the bag is liquid impervious. The layers may in particular comprise any material such as nonwovens or films. In a preferred embodiment of the present invention a laminate may be formed from a nonwoven layer and a film. The laminate can be formed by means known to the man skilled in the art.

Any nonwoven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag is preferably provided with a hydrophobic fibrous nonwoven layer. Such material layers present an uneven surface to the skin of the wearer and thus reduce significantly the problem of occlusion and greatly improves skin healthiness.

In one preferred embodiment of the present invention the bag comprises two layers. Preferably the outer layer (30) comprises said fibrous hydrophobic nonwoven layer and the inner layer (15) comprises a film.

In yet another preferred embodiment of the present invention, the bag (11) comprises three layers, preferably one film and two nonwoven layers. In an even more preferable embodiment the film is interposed between the two nonwoven layers. This sequence of layers results in a closed fibrous structure, which has a particularly pleasing sensation on contact with the skin of the wearer. In yet another preferred embodiment the inner layer comprises a film and the other two layers comprise nonwovens.

Typically, the nonwoven layer is treated with a surface active material, such as a fluorchemical or other hydrophobic finishings, to provide the requisite hydrophobicity. The nonwoven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The nonwoven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the nonwoven layer without rendering it greasy or oily to the tactile sense of the wearer. Additionally, surfactant material, including anionic, non-anionic, cationic and non-cationic surfactants, may be added to further enhance softness and surface smoothness.

Furthermore, the nonwoven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the nonwoven layer with a solid oil phase of cream formulation or to incorporate into the nonwoven layer an array of pressure- or thermal- or hydrorupturable capsules containing for example, baby oil.

In one embodiment of the present invention the bag may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag or may be secured to the inner layer of the bag (11). Any known techniques for securing absorbent material to nonwoven and film substrates may be used to secure the absorbent material to the inner layer of the bag. The absorbent material may also be arranged to have any desired shape or configuration (e.g., rectangular, oval, circular, etc.).

As shown in Figure 1 the bag (11) is provided with an aperture (21) whereby faecal matter is received from the body prior to storage within the bag cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction, most preferably the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange is attached to the bag by adhesive. Typically, the bag will be attached to the flange, towards the outer periphery of flange so as not to cause any obstruction for the entering faecal matter.

The flange may be provided in any size depending on the wearer group for which the device is intended. Similarly the flange may be provided in any shape and preferably has a symmetrical shape preferably comprising a plurality of lobes (13).

The flange comprises a garment facing portion (22) and a wearer facing portion (23). In an preferred embodiment these are two large, substantially flat surfaces, however, the flange (12) may also comprise projections designed to fit the perineal or coccygeal area of the wearer.

The flange (12) should be made of soft, flexible and malleable material to allow easy placement of the flange to the perianal area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/m², more preferably 50 g/m². Other thermoplastic foam materials, or other suitable plastics sheet materials having the described properties of such foams (i.e., softness, pliability, stretchability, and contractability) might also be used. Preferably, the material of garment facing surface (23) of the flange (12) may extend into the defined aperture area so as to form a skirt or flap of material which prevents unintentional adhesion of the surface edges of the flange (12) defining the aperture (21) to oneanother during use.

According to the present invention the faecal management device further comprises an attachment means to secure the device to the wearer. Such means include straps and more preferably comprises a body-compatible pressure sensitive adhesive (20) applied to the wearer facing portion (23) of the flange (12).

The adhesive (20) is preferably covered with a release means (not shown) in order to protect the adhesive (20) such as siliconized paper. The adhesive (20) can cover the entire wearer facing surface of the flange or more preferably have at least one, preferably two to six non-adhesive portions. These portions may be adhesive free or may contain inactivated or covered adhesives. As is evident from Figure 1, the adhesive is in one preferred embodiment not applied to the entire wearer facing surface area of the flange (12), so as to provide lobes (13) on either side of the flange (12) which are non-adhesive and can thereby serve to facilitate placement and removal of the device whilst avoiding contact with the adhesive. These lobes are however preferably also covered by the release means. Before application of the faecal management device (10) to the skin of the wearer, the release means if present is removed.

According to the present invention any medically approved water resistant pressure sensitive adhesive may be used to attach the device to the perianal area of the wearer, such as hydrocolloid adhesives and hydrogel adhesives. Particularly effective adhesives in providing the desired adhesive properties to secure the flange to the skin of the wearer at the sensitive perianal area, whilst allowing for relatively painless application and removal, are formed from crosslinking polymers with a plastisicer to form a 3-dimensional matrix.

The adhesive (20) can be applied to the wearer facing surface (23) of the flange (12) by any means known in the art such as slot coating, spiral, or bead application or printing. Typically the adhesive (20) is applied at a basis weight of from 20g/m² to 2500g/m², more preferably from 500g/m² to 2000g/m² most preferably from 700g/m² to 1500g/m² depending on the end use envisioned. For example for faecal management devices (10) to be used for babies the amount of adhesive (20) may be less than for faecal management devices designed for active adult incontinence sufferers.

### Detailed description of a diaper to be worn in combination with the faecal management device

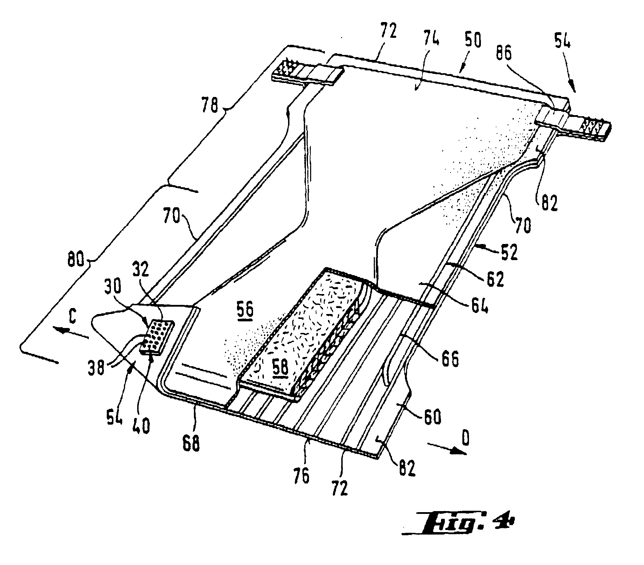

The faecal management device (10) of the present invention has been found to be particularly useful and beneficial when used in conjunction with a garment, or diaper (50), preferably a disposable diaper - refer to Figure 3. The faecal management device (10) is preferably first positioned in the perianal area of the wearer before the disposable diaper (50) is applied. In particular, the diaper (50) is positioned over the faecal management device (10) and fastened in a conventional manner around the body of the wearer. It has been found that, in addition, to providing excellent separation between urine and faecal material, the combined faecal management device (10) and diaper (50) system actually reduces skin irritation, which may at times occur, especially since the group of typical wearers includes the very old, the very young and the unhealthy wearers. In effect, the presence of the faecal management device (10) permits the formation of a separation layer between the skin of the wearer and the diaper (50), i.e. a part of the absorbent core (58) of the diaper (10). The diaper (50) can be of the conventional type (an embodiment of which is described below although not a limiting example by any means) or can be adapted to contain in an effective and comfortable manner the faecal management device (10) according to the teachings of the present invention.

As used herein, the term "disposable diapers" refers to articles which absorb and contain body extrudates; and more specifically, refers to articles which are placed against or in proximity to the body of the wearer to absorb and contain the various extrudates discharged from the body and which are intended to be discarded after a single use (i.e., they are not intended to be laundered or otherwise restored or reused) and, preferably, to be recycled, composted or otherwise disposed of in an environmentally compatible manner. As used herein, the term "diaper" refers to a garment generally worn by infants or incontinence sufferers that is drawn up between the legs and fastened about the waist of the wearer.

Figure 4 is a partially cut-away perspective view of a diaper (50) embodying the present invention prior to it being placed on the wearer over the faecal management device (10). As is visible from Figure 3, a preferred diaper (50) comprises a body portion (52) and a refastenable mechanical fastening device (54). A preferred body portion (52) comprises a liquid pervious topsheet (56), and absorbent core (58), a liquid impervious backsheet (60), and elastically contractible leg cuffs (62); each leg cuff (62) preferably comprising a side flap (64) and one or more elastic members (66). For simplicity purposes, only one elastic member (66) is shown in the side flap (64). While the topsheet (56), the absorbent core (58), the backsheet (60), the side flaps (64), and the elastic members (66) may be assembled in a variety of well-known configurations. A preferred disposable diaper configuration is shown and generally described in US 3,860,003, an even more preferred disposable diaper configuration is shown and generally described in WO 93/16669. In this preferred diaper configuration, the backsheet (60) is joined to the topsheet (56); the absorbent core (58) is positioned between the topsheet (56) and the backsheet (60); the side flaps (64) extend outwardly from and along each side edge of the absorbent core (58); and the elastic member (66) is operatively associated with each side flap (64).

Figure 3 shows the body portion (52) in which the topsheet (56) and the backsheet (60) are coextensive and have length and width dimensions generally larger than those of the absorbent core (58). The topsheet (56) is superposed on the backsheet (60) thereby forming the periphery (68) of the body portion (52).

The body portion (52) has an inside surface (74) and an outside surface (76). When a backsheet (60) is used, it typically forms the outside surface (76) of the body portion (52). The inside surface (74) is that surface of the diaper (50) opposite the outside surface (76) and in the embodiment shown is typically formed by the topsheet (56). In general, the inside surface (74) of the diaper (50) is that surface coextensive with the outside surface (76) and which is for the greater part in contact with the wearer when the diaper (50) is worn.

The absorbent core (58) of the body portion (52) may be any absorbent means which is generally compressible, conformable, non-irritating to the skin of the wearer, and capable of absorbing and retaining liquids such as urine and other certain bodily discharges. The absorbent core (58) may be manufactured in a variety of sizes and shapes (for example, rectangular, hour-glass, "T"-shaped, asymmetric, etc.) and from a wide variety of liquid absorbent materials commonly used in disposable diapers and other absorbent articles such as comminuted wood pulp which is generally referred to as airfelt. Examples of other suitable absorbent materials include creped cellulose wadding, meltblown polymers including coform, crosslinked cellulosic fibers, tissue including tissue wraps, absorbent foams, absorbent sponges, superabsorbent polymers, absorbent gelling materials, or any equivalent materials or combinations of materials. The configuration and construction of the absorbent core (58) may also be varied (for example, the absorbent core (58) may have varying caliper zones, hydrophilic gradients, superabsorbent gradients, or lower average density and lower average basis weight acquisition zones; or may comprise one or more layers or structures). Further, the size and absorbent capacity of the absorbent core (58) may be varied to accommodate wearers ranging from infants to adults.

The backsheet (60) is impervious to liquids (for example, urine) and is preferably manufactured from a thin plastic film, preferably a thermoplastic film, although other flexible liquid impervious materials may also be used. As used herein, the term "flexible" refers to materials which are compliant and which will readily conform to the general shape and contours of the human body. The backsheet (60) prevents the exudates absorbed and contained in the absorbent core (58) from soiling articles which are in contact with the diaper (50) such as undergarments and bedding. The backsheet (60) may thus comprise polymeric films such as thermoplastic films of polyethylene or polypropylene, or composite materials such as film-coated nonwoven material. Exemplary films are manufactured by Tredegar Industries, Inc. of Terre Haute, Ind., USA or BP-Chemical PlasTec, Rotbuchenstrasse 1, D-8000 München, Germany.

The backsheet (60) is preferably textured to provide a more clothlike appearance. Further, the backsheet (60) may also permit vapours to escape from the absorbent core (58) while still preventing exudates from passing through the backsheet (60) by, for example, being supplied with microapertures. The size of the backsheet (60) is dictated by the size of the absorbent core (58) and the exact diaper design selected.

The topsheet (56) of the diaper is compliant, soft feeling and non-irritating to the skin of the wearer. Further, the topsheet (56) is liquid pervious permitting liquids (for example, urine) to readily penetrate through its thickness. A suitable topsheet (56) may be manufactured from a wide range of materials, such as porous foams, reticulated foams, apertured films; or woven or nonwoven webs of natural fibres (for example, wood or cotton fibres) or from a combination of natural and synthetic fibres. Preferably, it is made of a material that isolates the skin of the wearer from liquids retained in the absorbent core (58).

There are a number of manufacturing techniques which may be used to manufacture the topsheet (56). For example, the topsheet (56) may be a nonwoven web of fibres. An exemplary topsheet (56) is carded and thermally bonded by means well-known to those skilled in the fabric art. A suitable topsheet (56) is manufactured by, for example, Veratec Inc., a division of International Paper Company, of Walpole, Mass., USA. A topsheet (56) particularly preferred for incontinence garments comprises a formed thermoplastic film.

## Claims

1. A faecal management device (10) comprising a bag (11), said bag (11) having an aperture and an anatomically-shaped flange (12) surrounding said aperture for adhesive attachment to the perianal area of wearer, said anatomically-shaped flange (12) being attached to said bag (11), said bag (11) comprising a wall material, said wall material having an outer surface (30) and an inner surface (15)
**characterised in that**
said outer surface (30) is provided with a fibrous layer, wherein said fibres are hydrophobic and have a wetting time of at least 1 hour.

2. A faecal management device according to claim 1, wherein said fibres have a wetting time of at least 6 hours.

3. A faecal management device (10) according to claims 1 and 2, wherein said outer surface (30) comprises a nonwoven layer.

4. Faecal management device (10) according to claim 1, wherein said inner surface (15) comprises a plastic film layer.

5. Faecal management device (10) according to claim 4, wherein said plastic film layer is permeable to air and to vapour.

6. Faecal management device (10) according to claims 3 and 4, wherein said nonwoven layer and said plastic film layer are in the form of a laminate.

7. Faecal management device (10) according to claim 1, wherein said wall material comprises 3 layers, wherein said inner surface is a nonwoven layer, said outer surface is said fibrous hydrophobic layer and wherein a plastic film layer is interposed between said inner and said outer surface.

8. A faecal management device (10) according to claim 7, wherein said inner surface nonwoven layer (15) is hydrophobic.

9. A faecal management device (10) according to any of the preceding claims, wherein said hydrophobic fibrous outer layer (30) is softened with agents to enhance softness.

10. A faecal management device (10) according to any of the preceding claims wherein said nonwoven layer is impregnated with a lotion.

11. The use of a faecal management device (10) according to any of the preceding claims in combination with a disposable diaper (50).

12. The use of a faecal management device (10) according to claim 11 whereby said faecal management device (10) is first positioned in the perianal area of the wearer and then said disposable diaper (50) is positioned over said faecal management device (10) and fastened in a conventional manner around body of said wearer.

13. The use of a faecal management device (10) according to claims 11 and 12 wherein said faecal management device (10) provides a separation layer between skin of said wearer and said disposable diaper (50).

## Patentansprüche

1. Fäkalienaufnahmevorrichtung (10), mit einer Tasche (11), wobei die Tasche (11) eine Öffnung und einen die Öffnung umgebenden anatomisch geformten Flansch (12) zur Klebebefestigung am perianalen Bereich eines Benutzers aufweist, wobei der anatomisch geformte Flansch (12) an der Tasche (11) befestigt ist, wobei die Tasche (11) ein Wandmaterial aufweist, das eine Außenoberfläche (30) und eine Innenoberfläche (15) hat, **dadurch gekennzeichnet, daß** die Auβenoberfläche (30) mit einer fasrigen Schicht ausgestattet ist, wobei die Fasern hydrophob sind und eine Nässungszeit von zumindest einer Stunde haben.

2. Fäkalienaufnahmevorrichtung nach Anspruch 1, bei welcher die Fasern eine Nässungszeit von zumindest sechs Stunden haben.

3. Fäkalienaufnahmevorrichtung (10) nach Anspruch 1 oder 2, bei welcher die Außenoberfläche (30) eine nicht-gewebte Schicht aufweist.

4. Fäkalienaufnahmevorrichtung (10) nach Anspruch 1, bei welcher die Innenoberfläche (15) eine Kunststoffolienschicht aufweist.

5. Fäkalienaufnahmevorrichtung (10) nach Anspruch 4, bei welcher die Kunststoffolienschicht luft- und dampfdurchlässig ist.

6. Fäkalienaufnahmevorrichtung (10) nach den Ansprüchen 3 und 4, bei welcher die nicht-gewebte Schicht und die Kunststoffolienschicht in Form eines Laminates sind.

7. Fäkalienaufnahmevorrichtung (10) nach Anspruch 1, bei welcher das Wandmaterial drei Schichten aufweist, wobei die Innenoberfläche eine nicht-gewebte Schicht ist, die Außenoberfläche die genannte fasrige hydrophobe Schicht ist und eine Kunststoffolienschicht zwischen der Innen- und der Außenoberfläche liegt.

8. Fäkalienaufnahmevorrichtung (10) nach Anspruch 7, bei welcher die nicht-gewebte Innenoberflächenschicht (15) hydrophob ist.

9. Fäkalienaufnahmevorrichtung (10) nach einem der vorhergehenden Ansprüche, bei welcher die hydrophobe fasrige Außenschicht (30) mit Stoffen zur Verbesserung der Weichheit weichgemacht ist.

10. Fäkalienaufnahmevorrichtung (10) nach einem der vorhergehenden Ansprüche, bei welcher die nicht-gewebte Schicht mit einer Lotion imprägniert ist.

11. Verwendung einer Fäkalienaufnahmevorrichtung (10) nach einem der vorhergehenden Ansprüche in Kombination mit einer Einwegwindel (50).

12. Verwendung einer Fäkalienaufnahmevorrichtung (10) nach Anspruch 11, wobei die Fäkalienaufnahmevorrichtung (10) zunächst im perianalen Bereich des Benutzers positioniert wird und dann die Einwegwindel (50) über der Fäkalienaufnahmevorrichtung (10) positioniert und in herkömmlicher Art und Weise rund um den Körper des Benutzers befestigt wird.

13. Verwendung einer Fäkalienaufnahmevorrichtung (10) nach den Ansprüchen 11 und 12, wobei die Fäkalienaufnahmevorrichtung (10) eine Trennschicht zwischen der Haut des Benutzers und der Einwegwindel (50) schafft.

## Revendications

1. Dispositif (10) de prise en charge des matières fécales, comprenant un sac (11), ledit sac (11) ayant une ouverture et une collerette de forme anatomique (12) entourant ladite ouverture et destinée à être attachée par adhésif à la région périnéale de l'utilisateur, ladite collerette de forme anatomique (12) étant attachée audit sac (11), ledit sac (11) comprenant un matériau de paroi, ledit matériau de paroi ayant une surface extérieure (30) et une surface intérieure (15),
**caractérisé en ce que** :
ladite surface extérieure (30) est pourvue d'une couche fibreuse, dans laquelle lesdites fibres sont hydrophobes et ont une durée de mouillage d'au moins 1 heure.

2. Dispositif (10) de prise en charge des matières fécales selon la revendication 1, dans lequel lesdites fibres ont une durée de mouillage d'au moins 6 heures.

3. Dispositif (10) de prise en charge des matières fécales selon les revendications 1 et 2, dans lequel ladite surface extérieure (30) comprend une couche non tissée.

4. Dispositif (10) de prise en charge des matières fécales selon la revendication 1, dans lequel ladite surface intérieure (15) comprend une couche de film plastique.

5. Dispositif (10) de prise en charge des matières fécales selon la revendication 4, dans lequel ladite couche de film plastique est perméable à l'air et à la vapeur.

6. Dispositif (10) de prise en charge des matières fécales selon les revendications 3 et 4, dans lequel ladite couche non tissée et ladite couche de film plastique se présentent sous la forme d'un stratifié.

7. Dispositif (10) de prise en charge des matières fécales selon la revendication 1, dans lequel ledit matériau de paroi comprend trois couches, où ladite surface intérieure est une couche non tissée, ladite surface extérieure est ladite couche hydrophobe fibreuse, et où une couche de film plastique est intercalée entre ladite surface intérieure et ladite surface extérieure.

8. Dispositif (10) de prise en charge des matières fécales selon la revendication 7, dans lequel ladite couche non tissée de surface intérieure (15) est hydrophobe.

9. Dispositif (10) de prise en charge des matières fécales selon l'une quelconque des revendications précédentes, dans lequel ladite couche extérieure fibreuse hydrophobe (30) est assouplie avec des agents destinés à améliorer la douceur.

10. Dispositif (10) de prise en charge des matières fécales selon l'une quelconque des revendications précédentes, dans lequel ladite couche non tissée est imprégnée d'une lotion.

11. Utilisation d'un dispositif (10) de prise en charge des matières fécales selon l'une quelconque des revendications précédentes en combinaison avec une couche jetable (50).

12. Utilisation d'un dispositif (10) de prise cn charge des matières fécales selon la revendication 11, où le dispositif(10) de prise en charge des matières fécales est tout d'abord positionné dans la région périnéale de l'utilisateur, puis ladite couche jetable (50) est positionnée par-dessus ledit dispositif (10) de prise en charge des matières fécales et fixée d'une manière classique autour du corps dudit utilisateur.

13. Utilisation d'un dispositif (10) de prise en charge des matières fécales selon les revendications 11 et 12, où ledit dispositif (10) de prise en charge des matières fécales constitue une couche de séparation entre la peau dudit utilisateur et ladite couche jetable (50).
